# EUROPEAN PATENT APPLICATION

(11) **EP 0 815 884 A1**
(43) Date of publication of application: **07.01.1998**
(21) Application number: 96905066.5
(22) Date of filing: 12.03.1996
(51) Int. Cl.: A61M 5/28, A61M 5/31, A61L 2/20

(54) **PREFILLED SYRINGE AND METHOD OF STERILIZING PREFILLED INJECTION**

(30) Priority: 13.03.1995 JP 52974/95
(71) Applicant: TAISHO PHARMACEUTICAL CO. LTD, Tokyo 171 (JP)
(72) Inventor: OKAMOTO, Kouichi, Toshima-ku, Tokyo 171 (JP); KOSANO, Yoshinori, Toshima-ku, Tokyo 171 (JP); KURINO, Masuo, Toshima-ku, Tokyo 171 (JP); SUGANUMA, Hiromi, Toshima-ku, Tokyo 171 (JP); SOTTA, Niroh, Toshima-ku, Tokyo 171 (JP); ARAI, Isamu, Toshima-ku, Tokyo 171 (JP)
(74) Representative: Weisert, Annekäte, Dipl.-Ing. Dr.-Ing.
(86) International application number: JP9600609
(87) International publication number: WO9628201

(57) **Abstract**

A prefilled syringe, which comprises a cylinder 1, at least one rubber stopper 5 and a plunger 6, both being inserted successively into the cylinder 1 apart from one another at a given distance, and a needle holder 2 communicated with the tip end of the cylinder 1 in a hermetically closed state and integrated with a needle-mounting part 2a at the tip end of a discharge cylinder part 20, on whose inner periphery are formed passages 25 in the flow direction of a parenteral solution in a rubber stopper 5-inserted state, the parenteral solution being prefilled between the rubber stopper 5 and the plunger 6 in the cylinder, wherein a means of hermetically closing the needle-mounting part 2a of the needle holder 2 is provided at the needle-mounting part 2a of the needle holder 2, and a method for sterilizing the prefilled parenteral solution in the prefilled syringe in such a hermetically closed state by heating. According to the prefilled syringe and the method for sterilizing the prefilled parenteral solution, when a parenteral solution prefilled in a syringe is to be sterilized by heating the syringe with steam or hot water, no permeation of steam into the discharge cylinder part of the needle holder occurs.

## Description

### TECHNICAL FIELD

The present invention generally relates to a prefilled syringe of such a type as to provide a parenteral solution as prefilled in a syringe to users, and a method for sterilizing a prefilled parenteral solution, and more particularly to a prefilled syringe in such a structure that the cylinder part of a needle holder is connected to the tip end of a cylinder in a hermetically closed state and a parenteral solution is prefilled between one or a plurality of rubber stoppers and a plunger, both being inserted successively into the syringe apart from one another at a given distance, and a method for sterilizing a prefilled parenteral solution therein in the above-mentioned manner.

### BACKGROUND ART

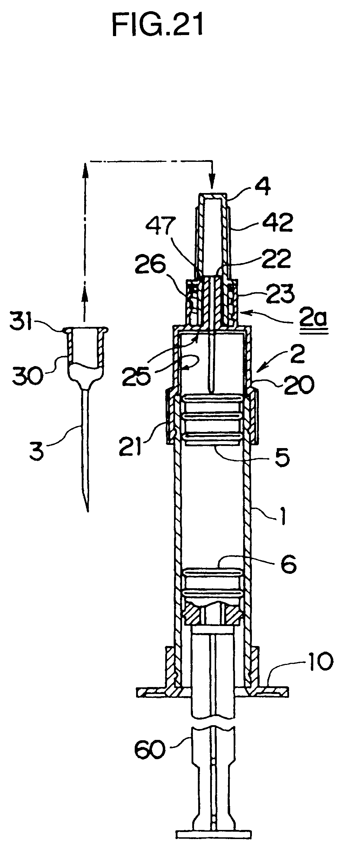

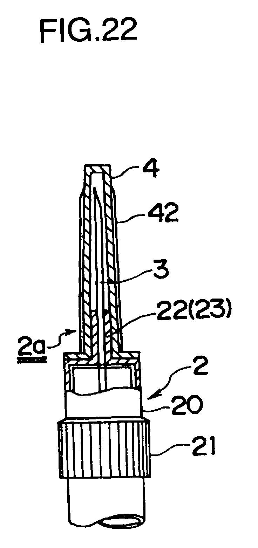

The structure of the conventional prefilled syringe and the conventional method for sterilizing a prefilled parenteral solution therein will be described below, referring to Figs. 21 and 22.

Fig. 21 shows the conventional, ordinary prefilled syringe, and Fig. 22 shows another type of the conventional prefilled syringe.

In Fig. 21, numeral 1 shows a hard glass cylinder and 2 an integrally molded needle holder of plastics such as polypropylene, polybutylene terephthalate, or the like.

Needle holder 2 is provided with a discharge cylinder part 20, a needle-mounting part 2a integrally formed with the tip end of the discharge cylinder part 20, and a joint part 21 integrally formed with the base end part of the discharge cylinder part 20. Needle holder 2 is connected to the tip end part of the cylinder 1 in a slipout-preventive manner and in a hermetical closed state by means of the joint part 21, whose outer periphery is knurled.

In this case, the needle-mounting part 2a comprises a central needle communication hub 22 and an outer peripheral needle-fixing part 23, each being integrally formed with the discharge cylinder part 20.

On the way of distribution routes or before their use, the needle-mounting part 2a of the syringe is covered with an integrally molded protective cap 4 of plastics such as polypropylene, polyethylene or the like. On the outer periphery of the protective cap 4 are formed an appropriate number of knurled thread-like projections 42 so as to facilitate finger gripping.

In this case, the protective cap 4 covers the needle communication hub 22.

When the protective cap 4 is put on the needle communication hub 22 from the tip end side, the cap 4 is brought into a slipout-preventive state through engagement in a projection-and-recess matching manner of cylindrical flange part 47 at the tip end part of the cap 4 with the inner periphery at the tip end part of the needle-fixing part 23, and thus is hardly pulled out from the hub 22 without application of some force. On the inner periphery at the tip end part of the protective cap 4 are formed spiral projections and recesses (not shown in the drawing) to form a so-called labyrinth structure capable of passing a gas or liquid but hard to pass microorganisms, etc. between the inner periphery of the protective cap 4 and the outer periphery of the needle communication hub 22.

The protective cap 4 is removed when the syringe is to be used, and a needle 3 is made to communicate with the needle communication hub 22.

In this case, a projection 31 is formed on the outer periphery at the end part of the needle base hub 30 of the needle 3, while spiral thread-like projections 26 to be engaged with the projection 31 are formed on the inner periphery of the needle-fixing part 23. When the needle base hub 30 is screwed into the needle-fixing part 23, the needle 3 can communicate with the throughhole of the needle communication hub 22.

A rubber stopper 5 and a plunger 6 are inserted into the cylinder 1 at a given distance, and a parenteral solution (not shown in the drawing) is filled between the rubber stopper 5 and the plunger 6.

The rubber stopper 5 and the plunger 6 are made of butyl rubber, butadiene rubber, these rubber laminated with ethylene tetrafluoride polymer (trademark: Teflon), or the like.

Numeral 60 shows a plunger rod screwed into the plunger 6, and 10 shows a finger grip mounted on the base end part of the cylinder 1 in a slipout-preventive manner. These members are molded from plastics such as polypropylene, polyethylene or the like.

By removing the protective cap 4 from the prefilled syringe, setting the needle 3 to the set position, and pushing the plunger 6 in the direction to the tip end of the cylinder 1 by the plunger rod 60, the rubber stopper 5 is pushed and moved in the direction to the tip end of the cylinder 1 into the discharge cylinder part 20 of the needle holder 2.

By further pushing the plunger 6 in that state, the parenteral solution existing between the rubber stopper 5 and the plunger 6 is injected from the needle 3 through groove-like passages 25 formed in the liquid flow direction on the inner wall of the discharge cylinder part 20.

In the prefilled syringe of Fig. 22, the needle communication hub 22 and the needle-fixing part 23, both constituting the needle-mounting part 2a, are in common, and the needle 3 is fixed to the needle communication hub 22, as inserted therein.

The protective cap 4 covers the needle communication hub 22 as put thereon by pressing.

A method for sterilizing the parenteral solution in the prefilled syringe of Fig. 21 will be described below, while outlining an assembling procedure of the syringe.

At first, a cylinder 1 including a finger grip 10, a needle holder 2 provided with a protective cap 4 (in case of Fig. 22, a needle 3 and the protective cap 4), the needle 3, a rubber stopper 5 and a plunger 6 are sterilized. These members are sterilized before their assembling or after assembling of all the members except the plunger 6, as shown in the drawing.

All the members except the plunger 6 are assembled together, as shown in the drawing, and after a parenteral solution is filled into the cylinder 1 from its base side, the plunger 6 is inserted into the cylinder 1.

The assembled syringe is placed in a sterilization chamber (not shown in the drawing), and the internal parenteral solution is sterilized, while keeping the syringe in an atmosphere heated by steam or hot water shower (80 - 130°C) for 5 to 30 minutes.

Then, the syringe is cooled (in many cases, cooled with water).

In the prefilled syringe, the needle-mounting part 2a of the needle holder 2 is covered with the protective cap 4, but is not hermetically closed by the needle-mounting part 2a.

Thus, while keeping the syringe in the atmosphere heated by steam or hot water shower as described above, steam or steam-containing hot water is permeated into the discharge cylinder part 20 of the needle holder 2 through fine clearances between the protective cap 4 and the needle-mounting part 2a, and condensed therein.

Most of water droplets condensed in the interiors are not simply dried out and remains there before the syringe is to be used, resulting in such a problem that the water droplets remaining in the discharge cylinder part 20 are mixed with the parenteral solution at the time of injection, and injected together into the living body.

### DISCLOSURE OF THE INVENTION

An object of the present invention is to provide a prefilled syringe free from permeation of steam or hot water into the discharge cylinder part of a needle holder when the parenteral solution prefilled in the syringe is sterilized by steam or hot water, and a method for sterilizing the parenteral solution.

The present prefilled syringe has the following structures so as to dissolve the above-mentioned problem.

According to an embodiment ① of the present invention, a prefilled syringe comprising at least one rubber stopper 5 and a plunger 6, both being inserted successively into a cylinder 1 apart from one another at a given distance, a needle holder 2 communicating with the tip end of the cylinder 1 in a hermetically closed state and integrated with a needle-mounting part 2a at the tip end part of a discharge cylinder part 20, on whose inner periphery are formed passages 25 in the flow direction of a parenteral solution in a rubber stopper 5-inserted state, the parenteral solution being prefilled between the rubber stopper 5 and the plunger 6 in the cylinder 1, characterized in that a means of hermetically closing the needle-mounting part 2a of the needle holder 2 is provided at the needle-mounting part 2a of the needle holder 2.

The term "hermetically closed" herein used has the same meaning as "hermetic" in the term "hermetic container" defined by Japanese Pharmacopoeia.

Another embodiment ② according to the present invention is characterized in that a protective cap 4 is put on the needle-mounting part 2a of the needle holder 2 in the syringe according to the embodiment ① of the present invention, and the outermost contact part between the protective cap 4 and the needle-mounting part 2a is hermetically closed by a rubber cover 7, which covers the contact part.

Other embodiment ③ according to the present invention is characterized in that in the syringe according to the embodiment ② of the present invention the entirety of the protective cap 4 is covered with the rubber cover 7. That is, the cover 7 is in a cap form.

Further embodiment ④ according to the present invention is characterized in that in the syringe according to the embodiment ① of the present invention a rubber protective cap 7a is put on the needle-mounting part 2a of the needle holder 2, and the needle-mounting part 2a is hermetically closed by the protective cap 7a.

Rubber materials for the rubber cover 7 and rubber protective cap 7a used in the embodiments ②, ③ and ④ of the present invention include, for example, natural and synthetic rubbers (butyl, butadiene, silicone, neoprene, polyurethane, fluorocarbon, acrylic, ethylene-propylene, nitrile-butadiene, isobutylene-isoprene, styrene-butadiene, polyvinylchloride), etc. The cover 7 and the protective cap 7a each have a rubber thickness of preferably 0.1 to 5 mm.

Further embodiment ⑤ according to the present invention is characterized in that in the syringe according to the embodiment ① of the present invention the protective cap 4 is put on the needle-mounting part 2a of the needle holder 2 and the contact part between the protective cap 4 and the needle holder 2 is sealed in a hermetically closed state by a sticky agent 4a serving also as a seal material.

The sticky agent 4a for use in the present invention includes, for example, a natural rubber-based sticky agent, a silicone-based sticky agent, a polybutene-based sticky agent, a styrene-isobutyrene rubber-based sticky agent, a polyisoprene rubber-based sticky agent, a styrene-isoprene-styrene rubber-based sticky agent, an acrylic sticky agent, a polyisobutyrene rubber-based sticky agent, a polyethylene-based hot melt-type sticky agent, etc. Preferable is a silicone-based sticky agent.

A further embodiment ⑥ according to the present invention is characterized in that in the syringe according to the embodiment ① of the present invention the protective cap 4 is put on the needle-mounting part 2a of the needle holder 2, the contact part between the protective cap 4 and the needle holder 2 is brought into adhesion in a hermetically closed state by an adhesive 4b, and an annular groove is formed on the outer periphery of the protective cap 4, the wall thickness of the protective cap 4 being made smaller at the groove position on the outer periphery of the needle-mounting part 2a by the annular groove.

The adhesive 4b for use in the present invention includes, for example, a natural rubber-based adhesive, a silicone-based adhesive, a polybutene-based adhesive, a styrene-isobutylene rubber-based adhesive, a polyisoprene rubber-based adhesive, a styrene-isoprene-styrene rubber-based adhesive, an acrylic adhesive, a polyisobutyrene rubber-based adhesive, a polyethylene-based hot melt type adhesive, etc. Preferable is a silicone-based adhesive.

Further embodiment ⑦ according to the present invention is such that in the syringe according to the embodiment ① of the present invention the needle-mounting part 2a of the needle holder 2 comprises a needle communication hub 22, which communicates with the discharge cylinder part 20 and a synthetic resin protective cap 4f is put on the needle communication hub 22 in a hermetically closed state.

In the syringe according to the embodiment ⑦, it is preferable that the needle communication hub 22 has a conical part 22a, whose diameter is made smaller towards the tip end part thereof, and has a male screw part 22b on the outer periphery at the base end thereof, while the protective cap 4f has a female screw part 40 to be screwed into the male screw part 22b on the inner periphery thereof and an inversely conical part 41 to be hermetically engaged with the conical part 22a with a pressing force to attain a hermetical closed state therebetween.

Further embodiment ⑧ according to the present invention is such that in the syringe according to the embodiment ① of the present invention the needle-mounting part 2a of the needle holder 2 comprises a needle communication hub 22, which communicates with the discharge cylinder part 20 and a short, cylindrical needle-fixing part 23, which is substantially concentrical with the needle communication hub 22, and the synthetic resin protective cap 4f to be put on the needle communication hub 22 is screwed into the needle-fixing part 23 on its inner periphery or outer periphery in a hermetically closed state.

Synthetic resins for use in the embodiments ⑦ and ⑧ of the present invention include, for example, polypropylene, polyethylene, polycarbonate, polyvinyl chloride, polyester, polystyrene, ethylene-vinyl acetate, ionomer, acrylic resin, polyurethane, ABS, polyacetal, acrylbutadiene-styrene, acrylstyrene, etc. Preferable are polypropylene, polycarbonate and polyester.

Further embodiment ⑨ according to the present invention is characterized in that in the syringe according to the embodiment ① of the present invention the protective cap 4 is put on the needle-mounting part 2a of the needle holder 2, and the contact part between the protective cap 4 and the needle holder 2 is sealed in a hermetically closed state by a seal material 4c.

The seal material 4c for use in the present invention includes, for example, a natural rubber-based sticky agent, a silicone-based sticky agent, a polybutene-based sticky agent, a styrene-isobutylene rubber-based sticky agent, a polyisoprene rubber-based sticky agent, a styrene-isoprene-styrene rubber-based sticky agent, an acrylic sticky agent, a polyisobutylene rubber-based sticky agent, a polyethylene-based hot melt type sticky agent, etc. Preferable is a silicone-based sticky agent.

Further embodiment 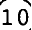 according to the present invention is characterized in that in the syringe according to the embodiment ① of the present invention the protective cap 4 is put on the needle-mounting part 2a of the needle holder 2, and the contact part between the protective cap 4 and the needle holder 2 is hermetically closed by a heat-shrunk shrink film 4d.

Materials for the shrink film 4d to be used in the present invention include, for example, polypropylene, polyethylene, polystyrene, polyvinylidene chloride, polyamide, ionomer, polyvinyl chloride, ethylene-vinyl acetate copolymer, polyester, nylon, silicone, fluorocarbon resin, etc. Preferable are polyvinyl chloride and polyester.

Further embodiment 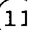 according to the present invention is characterized in that in the syringe according to the embodiment ① of the present invention the synthetic resin protective cap 4 is integrally formed with the needle-mounting part 2a at the tip end, and an annular groove is formed at the outer periphery at the base part of the protective cap 4, thereby making the wall thickness of the cap smaller at the groove position.

Synthetic resin for use in the embodiment 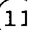 of the present invention includes, for example, polypropylene, polyethylene, polycarbonate, polyvinyl chloride, polyester, polystyrene, ethylene-vinyl acetate, ionomer, acrylic resin, polyurethane, ABS, polyacetal, acrylbutadiene-styrene, acrylstyrene, etc. Preferable are polypropylene, polyethylene and polycarbonate.

Further embodiment 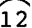 according to the present invention is characterized in that in the syringe according to the present invention the needle communication throughhole of the needle mounting part 2a is sealed into a hermetically closed state by a seal pin 4e inserted therein from the outside.

Materials for the seal pin to be used in the present invention include, for example, polypropylene, polyethylene, polycarbonate, polyvinyl chloride, polyester, polystyrene, ethylene-vinyl acetate, ionomer, acrylic resin, polyurethane, ABS, polyacetal, acrylbutadiene-styrene, acrylstyrene, etc. Preferable are polypropylene, polyethylene and polycarbonate.

In the sterilization treatment of a prefilled parenteral solution by heating with steam or hot water a syringe comprising a cylinder 1, at least one rubber stopper 5 and a plunger 6, both being inserted successively into the cylinder 1 apart from one another at a given distance, a needle holder 2 communicated with the tip end of the cylinder 1 in a hermetically closed state and integrated with a needle-mounting part 2a at the tip end of a discharge cylinder part 20, on whose inner periphery are formed passages 25 in the flow direction of a parenteral solution in a rubber stopper 5-inserted state, the parenteral solution being prefilled between the rubber stopper 5 and the plunger 6 in the cylinder 1, a method for sterilizing a prefilled parenteral solution according to further embodiment 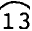 of the present invention is characterized by heating the syringe while keeping the needle-mounting part 2a of the needle holder 2 in a hermetically closed state. The present method for sterilization is carried out usually at 60 to 130°C for 1 to 130 minutes.

In the prefilled syringe according to the embodiment ① of the present invention, the needle-mounting part 2a of the needle holder 2 is hermetically closed and when the prefilled parenteral solution in the syringe is to be sterilized by heating the syringe with steam or hot water, no permeation of steam or hot water into the discharge cylinder part 20 of the needle holder 2 occurs.

In the prefilled syringe according to the embodiment ② of the present invention, the outermost contact part between the protective cap 4 put on the needle-mounting part 2a and the needle-mounting part 2a is hermetically closed by the rubber cover 7 for covering the contact part, and thus in the sterilization of the prefilled parenteral solution in the syringe by heating, as outlined above, no permeation of steam or hot water into the discharge cylinder part 20 of the needle holder 2 occurs.

When the syringe is to be used, the cover 7 and the protective cap 4 are removed therefrom.

In the prefilled syringe according to the embodiment ③ of the present invention, the entirety of the protective cap 4 is covered by the rubber cover 7, and thus when the syringe is to be used, the cover 7 can be removed therefrom easily together with the similar functions as those of the syringe according to the embodiment ② of the present invention.

In the prefilled syringe according to the embodiment ④ of the present invention, the needle-mounting part 2a is hermetically closed by the protective cap 7a put on the needle-mounting part 2a of the needle holder 2, and thus when the prefilled parenteral solution in the syringe is to be sterilized by heating, as outlined above, no permeation of steam or hot water into the discharge cylinder part 20 of the needle holder 2 occurs.

When the syringe is to be used, the protective cap 7a is removed from the needle-mounting part 2a.

In the prefilled syringe according to the embodiment ⑤ of the present invention, the contact part between the protective cap 4 put on the needle-mounting part 2a of the needle holder 2 and the needle holder 2 is sealed in a hermetically closed state by a sticky agent 4a serving as a seal material at the same time, and thus when the prefilled parenteral solution in the syringe is sterilized by heating, as outlined above, no permeation of steam or hot water into the discharge cylinder part 20 of the needle holder 2 occurs.

Furthermore, the sticky agent 4a serves as a seal material at the same time and is not a material capable of making permanent adhesion of one member to another, and thus when the syringe is to be used the protective cap 4 can be removed from the syringe without any partial breakage of the individual members which constitute the syringe.

In the prefilled syringe according to the embodiment ⑥ of the present invention, the contact part between the protective cap 4 put on the needle-mounting part 2a of the needle holder 2 and the needle holder 2 is brought into adhesion in a hermetically closed state by an adhesive 4b.

Thus, when the prefilled parenteral solution in the syringe is to be sterilized by heating, as outlined above, no permeation of steam or hot water into the discharge cylinder part 20 of the needle holder 2 occurs.

On the outer periphery of the protective cap 4 is formed the annular groove, which makes the wall thickness of the protective cap 4 smaller at the groove position thereof on the outer periphery of the needle-mounting part 2a, and thus when the syringe is to be used, the protective cap 4 can be easily detached and removed at the position of the groove 43.

In the prefilled syringe according to the embodiment ⑦ of the present invention, the synthetic resin protective cap 4f is engaged in a hermetically closed state with the outer periphery of the needle communication hub 22 by screwing, and thus when the prefilled parenteral solution is to be sterilized by heating, as outlined above, no permeation of steam or hot water into the discharge cylinder part 20 of the needle holder 2 occurs.

In the prefilled syringe according to the embodiment ⑧ of the present invention, the synthetic resin protective cap 4f put on the needle communication hub 22 is engaged in a hermetically closed state with the inner periphery or the outer periphery of the needle-fixing part 23 by screwing, and thus when the prefilled parenteral solution is to be sterilized by heating, as outlined above, no permeation of steam or hot water into the discharge cylinder part 20 of the needle holder 2 occurs.

In the prefilled syringe according to the embodiment ⑨ of the present invention, the contact part between the protective cap 4 and the needle holder 2 is sealed in a hermetically closed state by a seal material 4c, and thus when the prefilled parenteral solution is to be sterilized by heating, as outlined above, no permeation of steam or hot water into the discharge cylinder part 20 of the needle holder 2 occurs.

In the prefilled syringe according to the embodiment 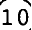 of the present invention, the contact part between the protective cap 4 and the needle holder 2 is hermetically closed by the heat-shrunk shrink film 4d, and thus when the prefilled parenteral solution is to be sterilized by heating, as outlined above, no permeation of steam or hot water occurs.

The shrink film 4d can be removed at the same time when the protective cap 4 is removed from the needle-mounting part 2a.

In the prefilled syringe according to the embodiment 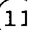 of the present invention, the synthetic resin protective cap 4 is integrally formed with the tip end of the needle-mounting part 2a, and thus when the prefilled parenteral solution is to be sterilized by heating, as outlined above, no permeation of steam or hot water into the discharge cylinder part 20 of the needle holder 2 occurs.

Furthermore, the annular groove, which makes the wall thickness of the protective cap 4 smaller at the groove position, is formed on the outer periphery at the base part of the protective cap 4, and thus when the syringe is to be used, the protective cap 4 can be easily detached and removed at the position of the groove 43.

In the prefilled syringe according to the embodiment 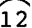 of the present invention, the needle communication throughhole of the needle-mounting part 2a is sealed in a hermetically closed state by the seal pin 4e inserted therein from the outside, and thus when the prefilled parenteral solution is to be sterilized by heating, as outlined above, no permeation of steam or hot water into the discharge cylinder part 20 of the needle holder 2 occurs.

The seal pin 4e is removed when the syringe is to be used.

In the method for sterilizing the prefilled solution according to the embodiment 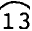 of the present invention, the syringe is heated by steam or hot water to sterilize the prefilled parenteral solution, while keeping the needle-mounting part 2a of the needle holder 2 in a hermetically closed state, and thus no permeation of steam or hot water into the discharge cylinder part 20 of the needle holder 2 occurs during the sterilization.

### BRIEF DESCRIPTION OF THE DRAWINGS

Figs. 1 to 17 are partial cross-sectional views showing embodiments of the present prefilled syringes.

Fig. 18 is a partially omitted front view of a group of syringes showing one embodiment of the present method for sterilization.

Fig. 19 is a partially cut-away, enlarged cross-sectional view along the arrow line A-A of Fig. 18.

Fig. 20 is a partial front view showing one embodiment of a double-prefilled syringe.

Figs. 21 and 22 are partial cross-sectional views showing examples of conventional prefilled syringes.

### BEST MODE FOR CARRYING OUT THE INVENTION

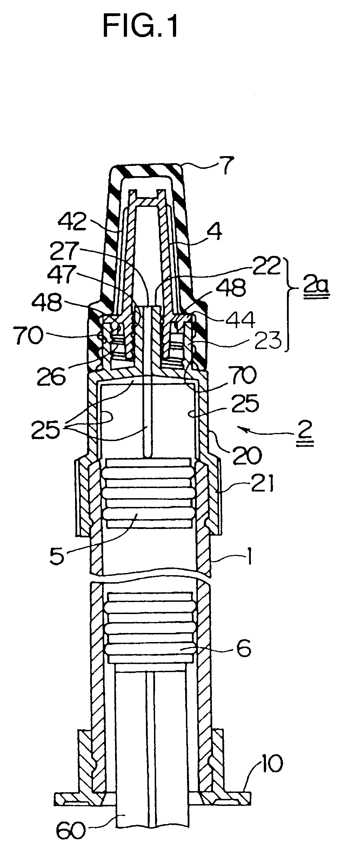

Preferable embodiments of the present prefilled syringe and method for sterilizing a prefilled parenteral solution will be described below, referring to Figs. 1 to 20.

In the following Examples, the same structural members as those of the conventional prefilled syringe described before in reference to Fig. 21 are identified with the same reference numerals and thus their description will be omitted.

### Example 1

Fig. 1 shows one embodiment of a prefilled syringe corresponding to the embodiments ② and ③ of the present invention.

In the syringe of this embodiment, needle-mounting part 2a of a needle holder 2 comprises a needle communication hub 22 having a needle communication throughhole 27, which communicates with a discharge cylinder part 20, and a cylindrical needle-fixing part 23 formed concentrically with the needle communication hub 22 and around the outer periphery thereof.

The needle communication hub 22 is covered with a protective cap 4 in a labyrinth structure, the cap having spiral projections and recesses 47 on the inner periphery at the lower part thereof. In the protective cap 4, a cylindrical flange part 48 on the outer periphery at the tip end thereof is engaged in a projection-and-recess matching manner with the inner periphery at the tip end part of a needle-fixing part 23 and thus the cap 4 cannot be slipped out from the needle-fixing part 23 without pulling-out with application of some force.

On the outer periphery of the protective cap 4 is formed a flange 44, which is in contact with the end part of the needle-fixing part 23 in a butted state.

The cylindrical needle-fixing part 23 is covered with a cap-formed butyl rubber cover 7 so as to cover the protective cap 4 and also cover the contact part between the flange 44 of the protective cap 4 and the needle-fixing part 23. The contact part between the flange 44 of the protective cap 4 and the needle-fixing part 23 is hermetically closed by the cover 7.

A plurality of annular projections and recesses 70 are formed at given distances in the axial direction on the inner periphery at the base end part of the cover 7, thereby facilitating insertion of the needle-fixing part 23 into the cover 7 and assuring the hermetically closed state at the contact part between the protective cap 4 and the needle-fixing part 23. It is preferable that the outer diameter of the needle-fixing part 23 is not less than the minimum inner diameter (minimum inner diameter at the annular projections) of the cover 7. The height of the annular projections is preferably 0.1 to 2.0 mm, while the number of annular projection is preferably 1 to 4.

When the syringe is to be used, the protective cap 4 and the cover 7 are removed from the needle-mounting part 2a, and a needle 3 is mounted on the needle-mounting part in the same manner as described in reference to Fig. 21.

100 samples each of prefilled syringe of this example and conventional syringe of Fig. 21, each of which contained a parenteral solution filled between a rubber stopper 5 and a plunger 6 in a cylinder 1 were manufactured.

The samples were divided into 4 respective groups each consisting of 50 samples, among which the two respective groups each of 50 samples were then kept in a hot water shower chamber at 110°C for 15 minutes, while the other two respective groups each of 50 samples were kept in a flowing steam atmosphere for 20 minutes to sterilize the prefilled parenteral solutions in the cylinders 1 by heating with hot water shower or steam.

Water droplets were found in discharge cylinder parts 20 of the needle holders 2 and also in the caps 4 in all 100 samples of the conventional prefilled syringes, whereas no water droplets were found in the corresponding parts in all 100 samples of the syringes of this Example as shown in Fig. 1.

In the syringe of this Example, when sterilization was carried out by heating with steam or hot water, as described above, permeation of steam or hot water into the discharge cylinder part 20 and the protective cap 4 could be prevented and no water droplets deposited in the corresponding parts. Thus, the embodiment of this Example was found most suitable to sterilization of prefilled parenteral solutions.

The cover 7 of this Example is mountable even if the needle-mounting part 2a comprises only a needle communication part 22. In this case, the functions of the cover 7 are the same as in case of the syringe of Fig. 1.

### Example 2

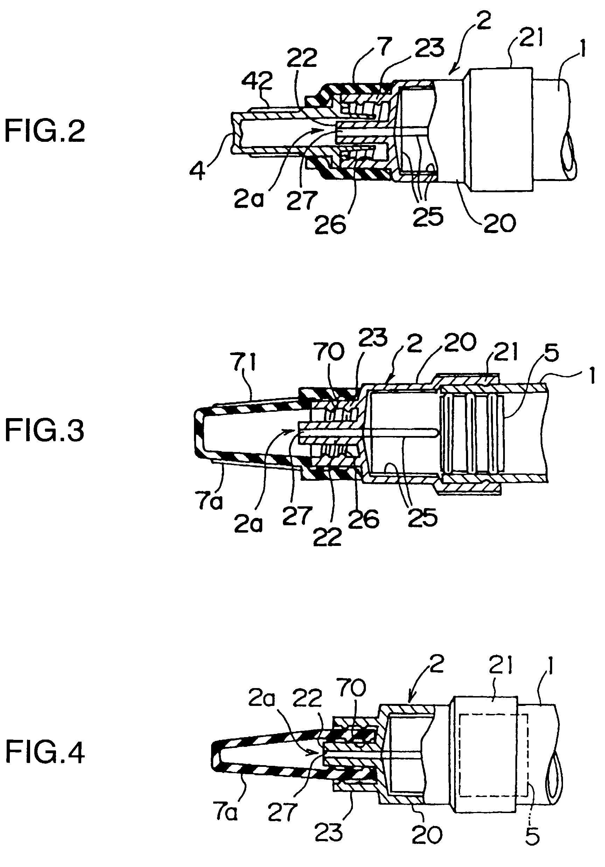

Fig. 2 shows a syringe of another embodiment corresponding to the embodiment ② of the present invention.

Butyl rubber cover 7 of this Example is not in a cap form as shown in Fig. 1, but the tip end part of a protective cap 4 is protruded from the cover 7.

Other structures, functions and effects of the syringe of this Example are substantially the same as those of Example 1, and thus their description is omitted.

### Example 3

Fig. 3 shows other example corresponding to the embodiment ④ of the present invention.

Needle-mounting part 2a of the syringe of this Example is substantially in the same structure as in Example 1, and a butyl rubber protective cap 7a is put on a cylindrical needle-fixing part 23 to hermetically close the needle-mounting part 2a.

On the inner periphery of the contact part between the protective cap 7a and the needle-fixing part 23 are formed a plurality of annular projections and recesses 70 in the same manner as those for the cover 7 of Example 1.

The protective cap 7a is removed therefrom when the syringe is to be used.

In the syringe of this Example, the needle-mounting part 2a is hermetically closed by the rubber protective cap 7a and thus when the prefilled parenteral solution is to be sterilized by heating with steam or hot water, no permeation of steam or hot water into the discharge cylinder part 20 of the needle holder 2 occurs. That is, no water droplets deposit in the discharge cylinder part 20 and also in the needle-mounting part 2a.

Other structures, functions and effects of the syringe of this Example are substantially the same as those of the syringe of Example 1 and thus their description is omitted.

### Example 4

Fig. 4 shows further embodiment corresponding to the embodiment ④ of the present invention.

In the syringe of this Example, a silicone rubber protective cap 7a is put on a needle communication hub 22 of a needle-mounting part 2a.

This structure is also applicable to a syringe whose needle-mounting part 2a comprises only a needle communication part 22, as shown in Fig. 22.

Other structures, functions and effects of the syringe of this Example are substantially the same as those of the syringe of Example 3, and thus their description is omitted.

### Example 5

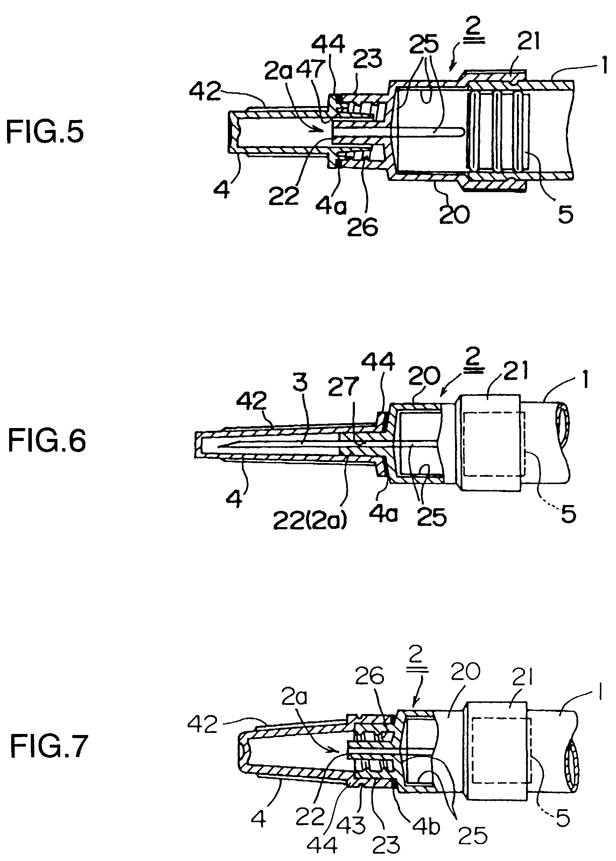

Fig. 5 shows a syringe embodiment corresponding to the embodiment ⑤ of the present invention.

In the syringe of this Example, a needle-mounting part 2a of a needle holder 2 comprises a needle communication hub 22 and a cylindrical needle-fixing part 23, and a protective cap 4 is put on the needle communication hub 22 in the same manner as in case of the syringe of Example 1.

The contact part between the protective cap 4 and the needle holder 2, that is, the contact part between the flange 44 of the protective cap 4 and the tip end part of the needle-fixing part 23, is brought into adhesion in a hermetically closed state, for example, by a silicone-based sticky agent 4a serving also as a seal material.

In the syringe of this Example, the contact part between the protective cap 4 and the needle holder 2 is sealed in a hermetically closed state by the sticky agent 4a serving also as a seal material, and thus when the prefilled parenteral solution is to be sterilized by heating with steam or hot water together with the syringe, no permeation of steam or hot water into the discharge cylinder 20 and the protective cap 4 occurs. That is, no water droplets deposit in these parts even if cooled.

Furthermore, the sticky agent 4a is no such an adhesive capable of effecting permanent adhesion, the protective cap 4 can be removed therefrom by pulling the cap 4 out therefrom.

Other structures, functions and effects of the syringe of this Example are substantially the same as those of the syringe of Example 1, and thus their description is omitted.

### Example 6

Fig. 6 shows further embodiment of a syringe corresponding to the embodiment ⑤ of the present invention.

In the syringe of this Example, a needle-mounting part 2a of a needle holder 2 comprises only a needle communication hub 22 serving also as a needle-fixing part, where a needle 3 is fixed to the needle communication hub 22 and a protective cap 4 is put thereon.

The contact part between the protective cap 4 and the needle holder 2 is brought into adhesion in a hermetically closed state by a sticky agent 4a serving also as a seal material.

Other structures, functions and effects of the syringe of this Example are substantially the same as those of the syringe of Example 5, and thus their description is omitted.

### Example 7

Fig. 7 shows a further syringe embodiment corresponding to the embodiment ⑥ of the present invention.

In the syringe of this Example, a needle-mounting part 2a of a needle holder 2 comprises a needle communication hub 22 and a cylindrical needle-fixing part 23, and a protective cap 4 is put on the needle-fixing part 23.

The contact part between the protective cap 4 and the needle holder 2, that is, the contact part between flange 44 of the protective cap 4 and the outer periphery of a discharge cylinder part 20 at the tip end, is brought into adhesion in a hermetically closed state by an adhesive 4b, and an annular groove 43 is formed on the outer periphery of the protective cap 4, the wall thickness of the protective cap 4 being made smaller at the groove position on the outer periphery of the needle-mounting part 2a by formation of the groove.

In the syringe of this Example, the contact part between the protective cap 4 and the needle holder 2 is brought into adhesion in a hermetically closed state by the adhesive 4b, and thus when the prefilled parenteral solution is to be sterilized by heating with steam or hot water together with the syringe, no permeation of steam or hot water into the discharge cylinder part 20 and the protective cap 4 occurs, that is, no water droplets deposit in these parts even when cooled.

The annular groove 43 is formed on the outer periphery of the protective cap 4 and the wall thickness of the protective cap 4 at the groove position is smaller, and thus the protective cap 4 can be easily removed from the needle-mounting part 2a by bending or twisting the protective cap 4 in one direction.

This structure of bringing the protective cap 4 having the annular groove 43 on its outer periphery into adhesion to the needle holder 2 by the adhesive 4b is applicable also to the syringe having the structure of Fig. 6.

Other structures, functions and effects of the syringe of this Example are substantially the same as those of the syringe of Example 1, and thus their description is omitted.

### Example 8

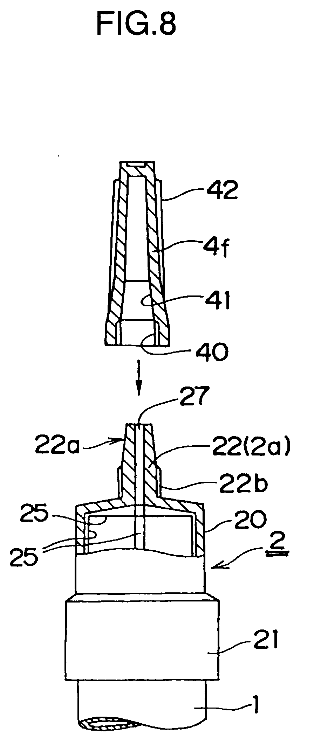

Fig. 8 shows further syringe embodiment corresponding to the embodiment ⑦ of the present invention.

In the needle-mounting part 2a of a needle holder 2 in the syringe of this Example, a needle communication hub 22, which communicates with a discharge cylinder part 20, serves as a needle-fixing part.

A synthetic resin protective cap 4f is put on the needle communication hub 22. The needle communication hub 22 has a conical surface 22a, whose diameter is made smaller towards the tip end, on the outer periphery near the tip end thereof, and a male thread part 22b is formed on the outer periphery near the tip end thereof at the same time.

On the inner periphery of the protective cap 4f, on the other hand, are formed a female thread part 40, which is screwed into the male thread part 22b in a hermetically closed state, and an inverse conical surface 41 so as to be hermetically fixed to the conical surface 22a in a pressed state, when the female thread part 40 is fastened to the male thread part 22b by screwing.

Into the needle communication hub 22, a needle (not shown in the drawing) may be inserted and fixed.

In the syringe of this Example, when the female thread part 40 of the protective cap 4f is fastened to the male thread part 22b of the needle communication hub 22 by screwing, the screw-engage parts is brought into a hermetically closed state, and also the inverse conical surface 41 on the inner periphery of the protective cap 4f is hermetically fixed to the conical surface 22a of the needle communication hub 22 in a hermetically closed state, and thus when the prefilled parenteral solution is to be sterilized by heating with steam or hot water together with the syringe, no permeation of steam or hot water into the discharge cylinder part 20 and the protective cap 4f occurs. That is, no water droplets deposit in these parts even if cooled.

Other structures, functions and effects of the syringe of this Example are substantially the same as those of the syringe of Example 1, and thus their description is omitted.

### Example 9

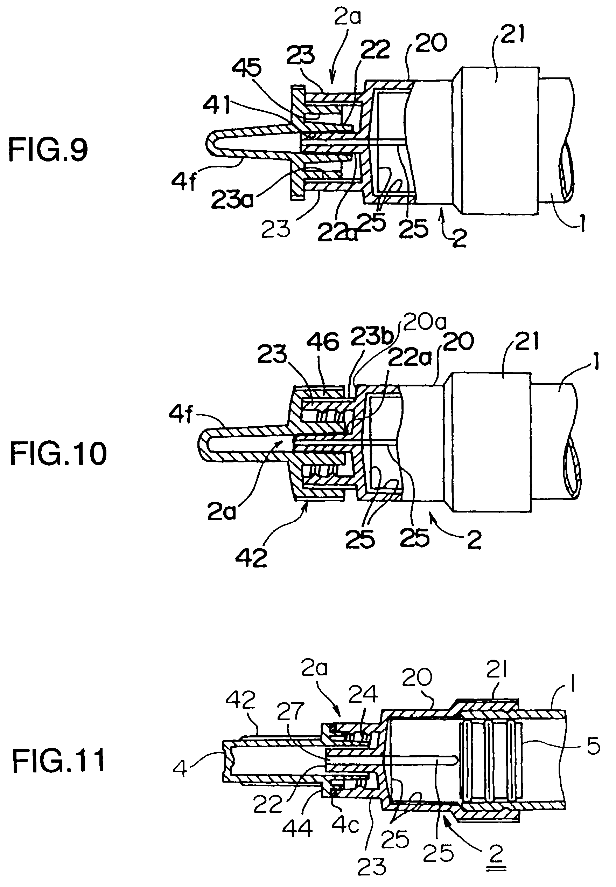

Fig. 9 shows a further syringe embodiment corresponding to the embodiment ⑧ of the present invention.

In the syringe of this Example, a needle-mounting part 2a of a needle holder 2 comprises a needle communication hub 22, which communicates with a discharge cylinder part 20, and a short, cylindrical needle-fixing part 23, which is substantially concentrical with the needle communication hub 22.

On the inner periphery of the needle-fixing part 23 is formed a female thread part 23a, and male thread part 45 of a synthetic resin protective cap 4f, which covers the needle communication hub 22, is engaged with the female thread part 23a in a hermetically closed state by screwing.

In the syringe of this Example, when the male thread part 45 of the protective cap 4 is fastened to the female thread part 23a of the needle-fixing part 23 by screwing, the screw-engaged parts are brought into a hermetically closed state, and thus when the prefilled parenteral solution is to be sterilized by heating with steam or hot water together with the syringe, no permeation of steam or hot water into the discharge cylinder part 20 and the protective cap 4f occurs. That is, no water droplets deposit in these parts even if cooled.

Other structures, functions and effects of the syringe of this Example are substantially the same as those of the syringe of Example 1, and thus their description is omitted.

### Example 10

Fig. 10 shows a further syringe embodiment corresponding to the embodiment ⑧ of the present invention.

The syringe of this Example is a modification of the syringe of Example 9, and a male screw part 23b is formed on the outer periphery of a needle-fixing part 23, whereas a female thread part 46, which is to be engaged with the male thread part 23b by screwing, is formed on a protective cap 4f.

Other structures, functions and effects of the syringe of this Example are substantially the same as those of the syringe of Example 9, and thus their description is omitted.

### Example 11

Fig. 11 shows a further syringe embodiment corresponding to the embodiment ⑨ of the present invention.

In the syringe of this Example, a needle-mounting part 2a of a needle holder 2 comprises a needle communication hub 22, which communicates with a discharge cylinder part 20, and a short, cylindrical needle-fixing part 23, which is substantially concentrical with the needle communication hub 22, as in substantially same manner as in the syringe of Example 1, and a protective cap 4 is weakly put on the needle communication hub 22 in a slipout-preventive manner.

The contact part between the protective cap 4 and the needle holder 2, that is, the contact part between the flange 44 of the protective cap 4 and the tip end part of the needle-fixing part 23, is sealed in a hermetically closed state by a seal material 4c such as a packing, etc.

In the syringe of this Example, the contact part between the tip end part of the needle-fixing part 23 and the flange 44 of the protective cap 4 is sealed in a hermetically closed state by the seal material 4c, and thus when the prefilled parenteral solution is sterilized with heating by steam or hot water together with the syringe, no permeation of steam or hot water into the discharge cylinder part 20 and the protective cap 4 occurs. That is, no water droplets deposit in these parts.

The seal material 4c can be also inserted between the end part of the protective cap 4f and shoulder part 20a of the discharge cylinder part 20 in the syringe of Fig. 10.

Other structures, functions and effects of the syringe of this Example are substantially the same as those of the syringe of Example 1, and thus their description is omitted.

### Example 12

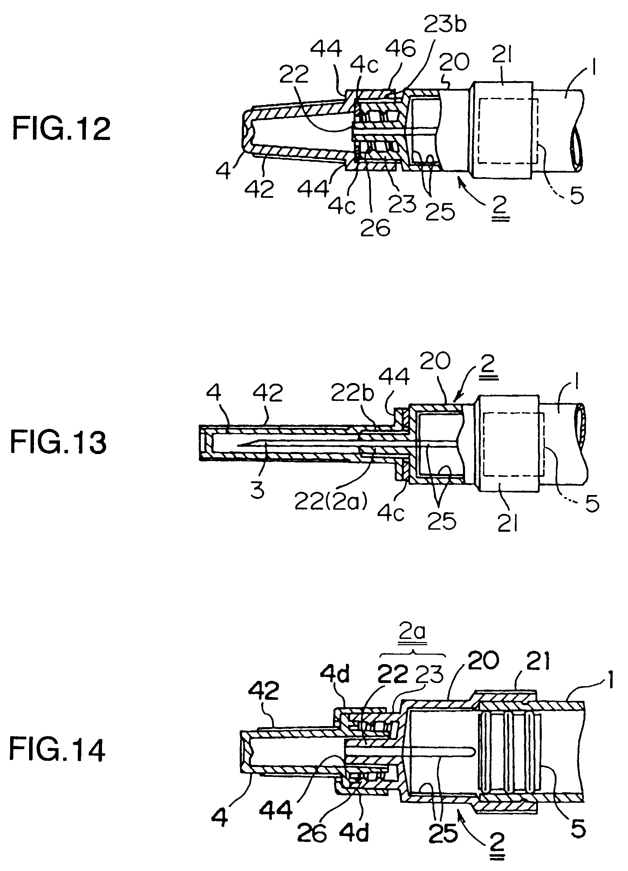

Fig. 12 shows a further syringe embodiment corresponding to the embodiment ⑨ of the present invention.

The syringe of this Example is a modification of the syringe of Example 11, where a male thread part 23b is formed on the outer periphery of a needle-fixing part 23, whereas a female thread part 46, which is to be engaged with the male thread part 23b by screwing, is formed on a protective cap 4.

The contact part between flange 44 of the protective cap 4 and the tip end of the needle-fixing part 23 is sealed in a hermetically closed state by a seal material 4c such as a packing, etc.

The protective cap 4 may be in such a structure, so as to be engaged with the inner periphery of the needle-fixing part 23 by screwing substantially in the same manner as in the syringe of Example 9.

In this Example, the seal material 4c can be inserted also between the end part of the protective cap 4 and the outer periphery of the discharge cylinder part 20 at the tip end thereof. In the needle-mounting part 2a may be fixed a needle.

Other structures, functions and effects of the syringe of this Example are substantially the same as those of the syringe of Example 11, and thus their description is omitted.

### Example 13

Fig. 13 shows a further syringe embodiment corresponding to the embodiment ⑨ of the present invention.

The syringe of this Example is a modification of the syringe of Example 11, where a needle-mounting part 2a consists of a needle communication hub 22 serving as a needle-fixing part, and a needle 3 is inserted and fixed to the needle communication hub 22.

A protective cap 4 is engaged with female thread part 22b formed on the outer periphery of the needle communication hub 22 by screwing, and the contact part between the protective cap 4 and a needle holder 2, that is, the contact part between flange 44 at the end part of the protective cap 4 and the outer periphery at the tip end of the discharge cylinder part 20, is sealed in a hermetically closed state by a seal material 4c.

Other structures, functions and effects of the syringe of this Example are substantially the same as those of the syringe of Example 11, and thus their description is omitted.

### Example 14

Fig. 14 shows a syringe embodiment corresponding to the embodiment 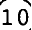 of the present invention.

In the syringe of this Example, a needle-mounting part 2a of a needle holder 2 comprises a needle communication part 22 and a needle-fixing part 23.

A protective cap 4 is put on the needle-fixing part 23 of the needle holder 2, and the contact part between the protective cap 4 and the needle holder 2, that is, the contact part between flange 44 of the protective cap 4 and the tip end part of the needle-fixing part 23 is hermetically closed by a heat-shrunk, shrink film 4d.

In the syringe of this Example, the outermost contact part between the protective cap 4 and the needle holder 2 is hermetically closed by the heat-shrunk, shrink film 4, and thus when the prefilled parenteral solution is to be sterilized by heating with steam or hot water together with the syringe, no permeation of steam or hot water into the discharge cylinder part 20 and the protective cap 4 occurs. That is, no water droplets deposit in these parts.

This Example can be applied to such a case that the needle-mounting part 2a comprises a needle communication hub 22 serving as a needle-fixing part, or also to such a case that the protective cap 4 is engaged with the inner periphery or the outer periphery of the needle-fixing part 23 by screwing, as shown in Fig. 9 or 10.

Other structures, functions and effects of the syringe of this Example are substantially the same as those of the syringe of Example 1, and thus their description is omitted.

### Example 15

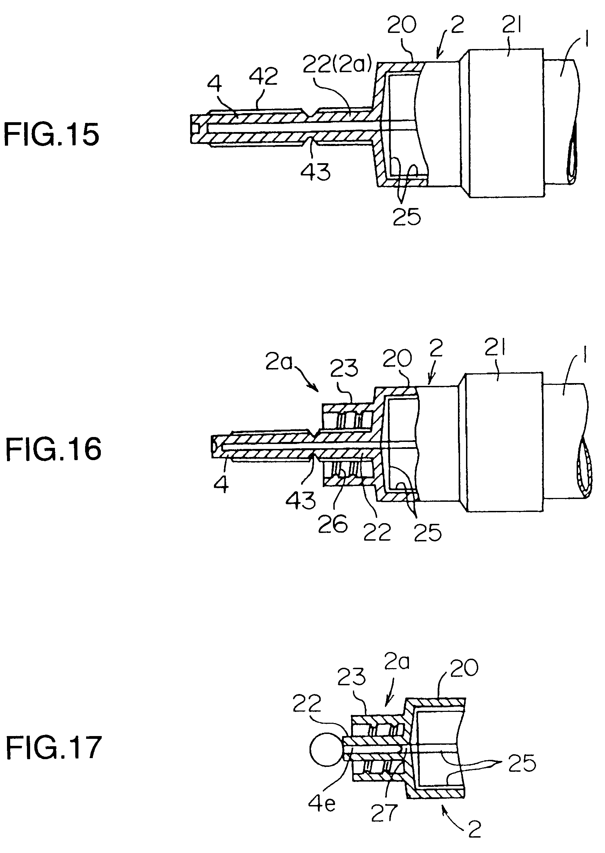

Fig. 15 shows a further syringe embodiment corresponding to the embodiment 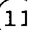 of the present invention.

In the syringe of this Example, a needle-mounting part 2a of a needle holder 2 comprises a needle communication hub 22 serving also as a needle-fixing part, and a protective cap 4 of the same synthetic resin as that of the hub 22 is integrally formed with the tip end of the needle communication hub 22.

On the outer periphery at the base end part of the protective cap 4 is formed an annular groove 43 so as to make the wall thickness of the protective cap 4 smaller at the groove position.

In the syringe of this Example, the needle communication hub 22 and the protective cap 4 are integrally formed from a synthetic resin, and thus when the prefilled parenteral solution is to be sterilized by heating with steam or hot water together with the syringe, no permeation of steam or hot water into the discharge cylinder 20 and the protective cap 4 occurs. That is, no water droplets deposit in these parts.

Furthermore, the annular groove 43 is formed on the outer periphery at the base end part of the protective cap 4, and the wall thickness is made smaller at the position of groove 43. Thus, by bending or twisting the protective cap 4 in one direction, the protective cap 4 can be easily removed from the needle-mounting part 2a.

Other structures, functions and effects of the syringe of this Example are substantially the same as those of the syringe of Example 1, and thus their description is omitted.

### Example 16

Fig. 16 shows another syringe embodiment corresponding to the embodiment 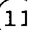 of the present invention.

In the syringe of this Example, a needle-mounting part 2a of a needle holder 2 comprises a needle communication hub 22 and a cylindrical needle-fixing part 23, and a protective cap 4 of the same synthetic resin as that of the needle communication hub 22 is integrally formed with the hub 22 at the tip end thereof. An annular groove 43 is formed on the outer periphery at the base end part of the protective cap 4, so as to make the wall thickness of the cap 4 smaller at the groove position.

In this Example, the protective cap 4 can be integrally formed with the tip end of the needle-fixing part 23.

Other structures, functions and effects of the syringe of this Example are substantially the same as those of the syringe of Example 15, and thus their description is omitted.

### Example 17

Fig. 17 shows syringe embodiment corresponding to the embodiment 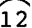 of the present invention.

In the syringe of this Example, needle-mounting part 2a of a needle holder 2 comprises a needle communication hub 22 and a cylindrical needle-fixing part 23, and needle communication throughhole 27 of the needle communication hub 22 is sealed in a hermetically closed state by a seal pin 4e inserted therein from the outside.

In the syringe of this Example, the needle communication throughhole 27 is sealed by the seal pin 4e, and thus when the prefilled parenteral solution is to be sterilized by heating with steam or hot water, no permeation of steam or hot water into a discharge cylinder part 20 occurs. That is, no water droplets deposit in the discharge cylinder part 20.

In this Example, the needle-mounting part 2a may comprise a needle communication hub 22 serving also as a needle-fixing part 23.

### Example 18

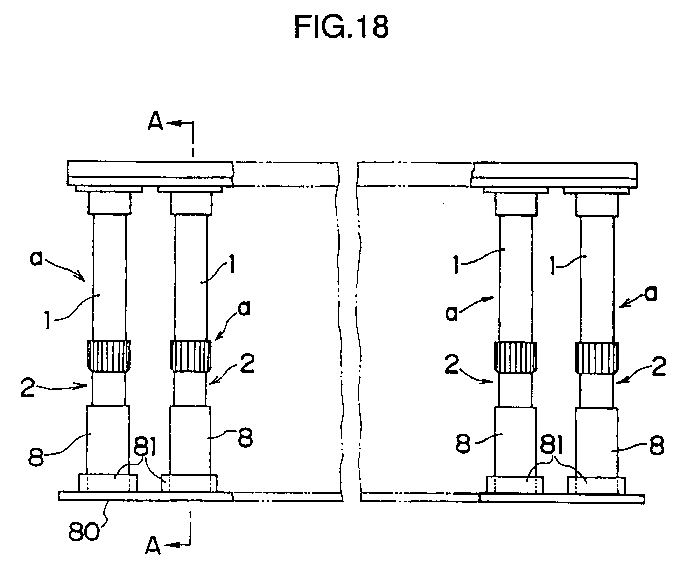

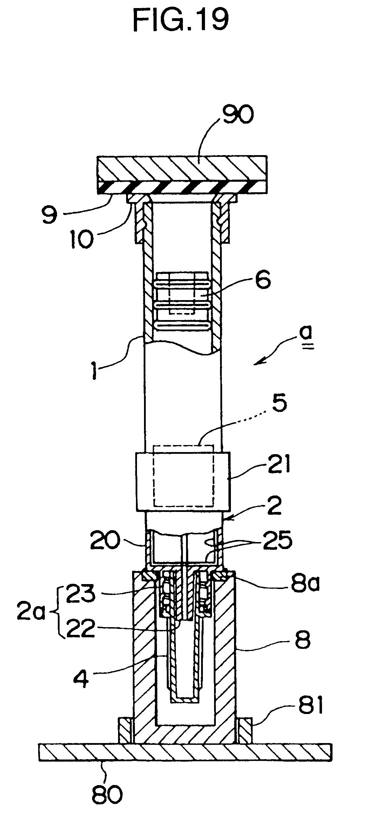

All the foregoing Examples show syringes and methods for sterilization to sterilize the prefilled parenteral solution by heating by providing a hermetically closing means at the necessary positions of the syringe. Figs. 18 and 19 show an embodiment of hermetically closing needle-mounting part 2a of a needle holder 2 in a syringe only at sterilization by heating.

In Figs. 18 and 19, symbol a shows a number of prefilled syringes, which are supported in an upside-down state by cup-formed receptors 8, respectively, arranged on a transfer plate 80.

Movement of all the receptors 8 is controlled by short, cylindrical guides 81 arranged on the transfer plate 80.

Each of the prefilled syringes a is substantially in the same structure as that of the syringe of Fig. 21, as shown in Fig. 19, and a protective cap 4 is put on needle communication hub 22 of a needle-mounting part 2a in a needle holder 2.

The prefilled syringes a are supported in a upside-down state so that the protective cap 4 and the entirety of the needle-mounting part 2a can be encased in the corresponding cup-formed receptors 8, and the shoulder part on the outer periphery at the tip end of a discharge cylinder part 20 is in contact with a packing or other seal ring 8a provided at the upper end on the inner periphery of the corresponding receptor 8.

The syringe a is in such a state that the plunger rod 60 of Fig. 21 is removed and is pressed down onto the receptor 8 under a given pressure exerted by an upper pressing member 90 through a rubber sheet 9.

Each of the syringes a supported by the corresponding receptors 8, as described above, are transferred into a steam-passing chamber or hot water shower chamber (not shown in the drawing) together with the transfer plate 80 and the pressing member 90.

Each of the syringes a is retained in the steam-passing chamber or hot water shower chamber (at about 110°C) for about 20 minutes, and the parenteral solution filled between a rubber stopper 5 and a plunger 6 in a cylinder 1 is sterilized at that time.

100 prefilled syringes a were arranged and supported on the transfer plate 80, as outlined and shown in the drawings, retained in the hot water shower chamber at about 110°C for about 20 minutes and then cooled. No water droplets were found in the discharge cylinder part 20 and the protective cap 4 in each of the syringes a.

According to the method for sterilization of this Example, the needle-mounting part 2a in the needle holder 2 is heated with steam or hot water in a sealed state by the seal ring 8a, and thus no permeation of steam or hot water into the discharge cylinder part 20 of the needle holder 2 and the protective cap 4 occurs in the sterilization step. That is, no water droplets deposit in the discharge cylinder part 20.

Furthermore, no permeation of steam or hot water into the region above the plunger 6 in the cylinder 1 occurs, and thus that region undergoes no wetting, making the successive treatment much easier.

### Other Example

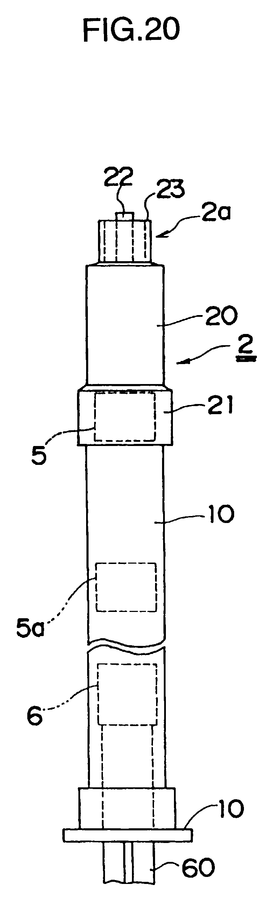

Fig. 20 shows the structure of another prefilled syringe. Into cylinder 1 of the syringe are inserted rubber stoppers 5 and 5a, and a plunger 6, apart from one another at given distances. Between the rubber stoppers 5 and 5a is filled a parenteral solution or a drug (no limited to a liquid) and between the rubber stopper 5a and the plunger 6 is filled a parenteral solution.

That is, two different parenteral solutions are filled in two spaces in the cylinder 1 of the syringe of Fig. 20, respectively, and by pushing plunger 6 in an injection director by a plunger rod 60, the rubber stoppers 5 and 5a move towards the discharge cylinder part 20 of a needle holder 2, and the first rubber stopper 5 enters the discharge cylinder part 20.

By further pushing the plunger 6 in that state, the parenteral solution between the rubber stoppers 5 and 5a is injected, and then the rubber stopper 5a also enters the discharge cylinder part 20. By further pushing the plunger 6, the parenteral solution between the rubber stopper 5a and the plunger 6 is injected.

The structures and methods for sterilization of syringes of each of the foregoing embodiments of the present invention can be applied to syringes of double prefilled type as in Fig. 20.

When a drug is filled between the rubber stoppers 5 and 5a in the syringe of Fig. 20, the rubber stopper 5, the drug and the rubber stopper 5a enter the discharge cylinder part 20 by pushing the plunger 6. By further pushing the plunger 6 in that state, the parenteral solution between the rubber stopper 5a and the plunger 6 flows into the discharge cylinder part 20 through passages 25. The drug is dissolved by the parenteral solution, and thus the parenteral solution containing the drug as dissolved can be injected.

According to the embodiment ① of the present invention, a needle-mounting part 2a of a needle holder 2 is hermetically closed, and when a parenteral solution prefilled in a syringe is to be heat-sterilized by heating the syringe with steam or hot water, no permeation of steam or hot water into discharge cylinder part 20 of the needle holder 2 occurs. That is, no water droplets deposit in the discharge cylinder part 20 even if cooling, this embodiment is suitable for sterilization of a prefilled parenteral solution by heating.

According to the embodiment ② of the present invention, a needle-mounting part 2a is hermetically closed by a rubber cover 7, which covers the outermost contact part between a protective cap 4 put on the needle-mounting part 2a and the needle-mounting part 2a, and thus when the parenteral solution in a syringe is to be sterilized by heating, as outlined above, no permeation of steam or hot water into discharge cylinder part 20 of a needle holder 2 occurs. Thus, the embodiment is suitable for sterilization of a prefilled parenteral solution by heating.

According to the embodiment ③ of the present invention, the entirety of a protective cap 4 is covered with a rubber cover 7, and thus the same effects as those of the syringe according to the embodiment ② of the present invention as well as another effect of easy removal of the cover 7 when the syringe is to be used can be obtained.

According to the embodiment ④ of the present invention, a rubber protective cap 7a put on needle-mounting part 2a of a needle holder 2 hermetically closes the needle-mounting part 2a, and thus when the parenteral solution in a syringe is to be sterilized by heating, as outlined above, no permeation of steam or hot water into discharge cylinder part 20 of the needle holder 2 occurs. Thus, the embodiment is suitable for sterilization of a prefilled parenteral solution by heating.

According to the embodiment ⑤ of the present invention, the contact part between a protective cap 4 put on needle-mounting part 2a of a needle holder 2 and the needle holder 2 is sealed in a hermetically closed state by a sticky agent 4a serving also as a seal material, and thus when the prefilled parenteral solution prefilled in a syringe is to be sterilized by heating, as outlined above, no permeation of steam or hot water into discharge cylinder part 20 of the needle holder 2 occurs. That is, the embodiment is suitable for sterilization of a prefilled parenteral solution by heating.

Furthermore, the sticky agent 4a serves as a seal material, and is no such a material as to ensure permanent adhesion of one member to another, and thus when the syringe is to be used, the protective cap 4 can be removed from the syringe without any breakage of parts of members which constitute the syringe.

According to the embodiment ⑥ of the present invention, the contact part between a protective cap 4 put on needle-mounting part 2a of a needle holder 2 and the needle holder 2 is brought into adhesion in a hermetically closed state by an adhesive 4b, and thus when the parenteral solution in a syringe is to be sterilized by heating, as outlined above, no permeation of steam or hot water into discharge cylinder part 20 of the needle holder 2 occurs. That is, the embodiment is suitable for sterilization of a prefilled parenteral solution by heating.

An annular groove 43 is formed on the outer periphery of the protective cap 4, thereby making the wall thickness of the protective cap 4 smaller at the groove position on the outer periphery of the needle-mounting part 2a, and thus when the syringe is to be used, the protective cap 4 can be easily detached and removed at the position of groove 43.

According to the embodiments ⑦ and ⑧ of the present invention, a synthetic resin protective cap 4f is engaged in a hermetically closed state with a needle-mounting part 2a by screwing, and thus when the parenteral solution in the syringe is to be sterilized, as outlined above, no permeation of steam or hot water into discharge cylinder part 20 of a needle holder 2 occurs. That is, the embodiments are suitable for sterilization of a prefilled parenteral solution.

According to the embodiment ⑨ of the present invention, the contact part between a protective cap 4 and a needle holder 2 is sealed in a hermetically closed state by a seal material 4c, and thus when the prefilled parenteral is to be sterilized by heating, as outlined above, no permeation of steam or hot water into discharge cylinder part 20 of the needle holder 2 occurs. That is, the embodiment is suitable for sterilization of a prefilled parenteral solution by heating.

According to the embodiment 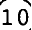 of the present invention, the contact part between a protective cap 4 and a needle holder 2 is hermetically closed by a heat-shrunk, shrink film 4d, and thus when the prefilled parenteral solution is to be sterilized by heating, as outlined above, no permeation of steam or hot water into discharge cylinder part 20 of the needle holder 2 occurs. That is, the embodiment is suitable for sterilization of a prefilled parenteral solution by heating.

According to the embodiment 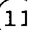 of the present invention, a synthetic resin protective cap 4 is integrally formed with the tip end of a needle-mounting part 2a, and thus when the prefilled parenteral solution is to be sterilized by heating, as outlined above, no permeation of steam or hot water into discharge cylinder part 20 of a needle holder occurs. That is, the embodiment is suitable for sterilization of a prefilled parenteral solution by heating.

Furthermore, an annular groove is formed on the outer periphery at the base end part of the protective cap 4, thereby making the wall thickness of the protective cap 4 smaller at the groove position, and thus when the syringe is to be used, the protective cap 4 can be easily detached and removed at the position of groove 43.

According to the embodiment 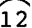 of the present invention, the needle communication throughhole of a needle-mounting part 2a is sealed in a hermetically closed state by a seal pin 4e inserted therein from the outside, and thus when the prefilled parenteral solution is to be sterilized by heating, as outlined above, no permeation of steam or hot water into discharge cylinder part 20 of a needle holder 2 occurs. That is, the embodiment is suitable for sterilization of a prefilled parenteral solution by heating.

According to the embodiment 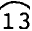 of the present invention, in sterilization of a prefilled parenteral solution by heating with steam or hot water, the syringe is heated while hermetically closing needle-mounting part 2a of a needle holder 2, and thus no permeation of steam or hot water into discharge cylinder part 20 of the needle holder 2 occurs. That is, no water droplets deposit in the discharge cylinder part 20 of a syringe.

## Claims

1. A prefilled syringe, which comprises a cylinder 1, at least one rubber stopper 5 and a plunger 6, both being inserted successively into the cylinder 1 apart from one another at a give distance, and a needle holder 2 communicated with the tip end of the cylinder 1 in a hermetically closed state and integrated with a needle-mounting part 2a at the tip end of a discharge cylinder part 20, on whose inner periphery are formed passages 25 in the flow direction of a parenteral solution in a rubber stopper 5-inserted state, the parenteral solution being prefilled between the rubber stopper 5 and the plunger 6 in the cylinder 1, wherein a means of hermetically closing the needle-mounting part 2a of the needle holder 2 is provided at the needle-mounting part 2a of the needle holder 2.

2. A prefilled syringe according to Claim 1, wherein the hermetically closing means comprises a protective cap 4 put on the needle-mounting part 2a of the needle holder 2 and a rubber cover 7, which covers the outermost contact part between the protective cap 4 and the needle-mounting part 2a, thereby hermetically closing the outermost contact part.

3. A prefilled syringe according to Claim 2, wherein the entirety of the protective cap 4 is covered by the rubber cover 7.

4. A prefilled syringe according to Claim 1, wherein the hermetically closing means comprises a rubber protective cap 7a put on the needle-mounting part 2a, thereby hermetically closing the needle-mounting part 2a of the needle holder 2.

5. A prefilled syringe according to Claim 1, wherein the hermetically closing means is provided by putting a protective cap 4 on the needle-mounting part 2a of the needle holder 2 and sealing the contact part between the protective cap 4 and the needle holder 2 by a sticky agent 4a serving also as a seal material in a hermetically closed state.

6. A prefilled syringe according to Claim 1, wherein the hermetically closing means is provided by engaging a synthetic resin protective cap 4f with the outer periphery of a needle communication hub 22 by screwing in a hermetically closed state, the needle-mounting part 2a of the needle holder 2 comprising the needle communication hub 22, which communicates with the discharge cylinder part 20.

7. A prefilled syringe according to Claim 1, wherein the hermetically closing means is provided by engaging a synthetic resin protective cap 4f to be put on a needle communication hub 22 with the inner periphery or outer periphery of the needle-fixing part 23 by screwing in a hermetically closed state, the needle-mounting part 2a of the needle holder 2 comprising the needle communication hub 22, which communicates with the discharge cylinder part 20, and the needle-fixing part 23 which is short, cylindrical and substantially concentrical with the needle communication hub 22.

8. A prefilled syringe according to Claim 1, wherein the hermetically closing means comprises a protective cap 4 put on the needle-mounting part 2a of the needle holder 2 and a heat-shrunk, shrink film 4d provided at the contact part between the protective cap 4 and the needle holder 2.

9. A method for sterilizing a prefilled parenteral solution, which comprises heating with steam or hot water a syringe comprising a cylinder 1, at least one rubber stopper 5 and a plunger 6, both being inserted successively into the cylinder 1 apart from one another at a given distance, and a needle holder 2 communicated with the tip end of the cylinder 1 in a hermetically closed state and integrated with a needle-mounting part 2a at the tip end of a discharge cylinder part 20, on whose inner periphery are formed passages 25 in the flow direction of a parenteral solution in a rubber stopper 5-inserted state, the parenteral solution being prefilled between the rubber stopper 5 and the plunger 6 in the cylinder 1, characterized by heating the syringe while keeping the needle-mounting part 2a of the needle holder 2 in a hermetically closed state.
